# EUROPEAN PATENT SPECIFICATION

(11) **EP 1 621 143 B1**
(45) Date of publication and mention of the grant of the patent: **07.01.2009**
(21) Application number: 05254694.2
(22) Date of filing: 27.07.2005
(51) Int. Cl.: A61B 17/072, A61B 17/28

(54) **Surgical instrument incorporating an electrically actuated pivoting articulation mechanism**
Mit elektrisch aktuierter Gelenkeinrichtung versehenes, chirurgisches Instrument
Instrument chirurgicale avec articulation à mise en action électrique

(30) Priority: 28.07.2004 US 591694 P; 31.03.2005 US 96096
(43) Date of publication of application: 01.02.2006
(73) Proprietor: ETHICON ENDO-SURGERY, INC., Cincinnati, Ohio 45242 (US)
(72) Inventor: Shelton IV, Frederick, Hillsboro Ohio 45133 (US)
(74) Representative: Tunstall, Christopher Stephen

(56) References cited:
- US-A- 5 673 841
- US-A1- 2003 065 358
- US-A1- 2003 069 474

## Description

### Field of the Invention

The present invention relates in general to surgical instruments that are suitable for endoscopically inserting an end effector (e.g., endocutter, grasper, cutter, staplers, clip applier, access device, drug/gene therapy delivery device, and an energy device using ultrasound, RF, laser, etc.) to a surgical site, and more particularly to such surgical instruments with an articulating shaft.

### Background of the Invention

Endoscopic surgical instruments are often preferred over traditional open surgical devices since a smaller incision tends to reduce the post-operative recovery time and complications. Consequently, significant development has gone into a range of endoscopic surgical instruments that are suitable for precise placement of a distal end effector at a desired surgical site through a cannula of a trocar. These distal end effectors engage the tissue in a number of ways to achieve a diagnostic or therapeutic effect (e.g., endocutter, grasper, cutter, staplers, clip applier, access device, drug/gene therapy delivery device, and energy device using ultrasound, RF, laser, etc.).

Positioning the end effector is constrained by the trocar. Generally these endoscopic surgical instruments include a long shaft between the end effector and a handle portion manipulated by the clinician. This long shaft enables insertion to a desired depth and rotation about the longitudinal axis of the shaft, thereby positioning the end effector to a degree. With judicious placement of the trocar and use of graspers, for instance, through another trocar, often this amount of positioning is sufficient. Surgical stapling and severing instruments, such as described in U.S. Pat. No. 5,465,895, are an example of an endoscopic surgical instrument that successfully positions an end effector by insertion and rotation.

More recently,US-A1-2004232191, "SURGICAL STAPLING INSTRUMENT INCORPORATING AN E-BEAM FIRING MECHANISM" to Shelton et al., filed on 20 May 2003. describes an improved "E-beam" firing bar for severing tissue and actuating staples. Some of the additional advantages include affirmatively spacing the jaws of the end effector, or more specifically a staple applying assembly, even if slightly too much or too little tissue is clamped for optimal staple formation. Moreover, the E-beam firing bar engages the end effector and staple cartridge in a way that enables several beneficial lockouts to be incorporated.

Depending upon the nature of the operation, it may be desirable to further adjust the positioning of the end effector of an endoscopic surgical instrument. In particular, it is often desirable to orient the end effector at an axis transverse to the longitudinal axis of the shaft of the instrument. The transverse movement of the end effector relative to the instrument shaft is conventionally referred to as "articulation". This is typically accomplished by a pivot (or articulation) joint being placed in the extended shaft just proximal to the staple applying assembly. This allows the surgeon to articulate the staple applying assembly remotely to either side for better surgical placement of the staple lines and easier tissue manipulation and orientation. This articulated positioning permits the clinician to more easily engage tissue in some instances, such as behind an organ. In addition, articulated positioning advantageously allows an endoscope to be positioned behind the end effector without being blocked by the instrument shaft.

Approaches to articulating a surgical stapling and severing instrument tend to be complicated by integrating control of the articulation along with the control of closing the end effector to clamp tissue and fire the end effector (i.e., stapling and severing) within the small diameter constraints of an endoscopic instrument. Generally, the three control motions are all transferred through the shaft as longitudinal translations. For instance, U.S. Pat. No. 5,673,840 discloses an accordion-like articulation mechanism ("flex-neck") that is articulated by selectively drawing back one of two connecting rods through the implement shaft, each rod offset respectively on opposite sides of the shaft centerline. The connecting rods ratchet through a series of discrete positions.

Another example of longitudinal control of an articulation mechanism is U.S. Pat. No. 5,865,361 that includes an articulation link offset from a camming pivot such that pushing or pulling longitudinal translation of the articulation link effects articulation to a respective side. Similarly, U.S. Pat. No. 5,797,537 discloses a similar rod passing through the shaft to effect articulation.

In co-pending and commonly owned US-A1-2005006434"SURGICAL INSTRUMENT INCORPORATING AN ARTICULATION MECHANISM HAVING ROTATION ABOUT THE LONGITUDINAL AXIS" to Frederick E. Shelton IV et al, a rotational motion is used to transfer articulation motion as an alternative to a longitudinal motion.

While these mechanically communicated articulation motions have successfully enabled an endoscopic surgical stapling and severing instrument to articulate, development trends pose numerous challenges and barriers to entry into the market. Conflicting design objects include a shaft of as small a diameter as possible to reduce the size of the surgical opening yet with sufficient strength to perform the several motions (e.g., closing, firing, articulation, rotation, etc.)

Consequently, a significant need exists for an articulating surgical instrument that incorporates an articulation mechanism that requires less mechanical mechanisms passing through the shaft of the instrument.

US-A- 20030065358 discloses a surgical instrument in which an EAP actuator may replace tendon wire actuation of a pivoting articulation joint, as in the preamble to present claim 1.

### Brief Summary of the Invention

The invention overcomes the above-noted and other deficiencies of the prior art by providing a surgical instrument having an end effector, an elongate shaft and a pivoting articulation joint, as in appended claim 1.

One aspect of the invention provides the surgical instrument has an articulating shaft attached between a handle and an end effector. An electroactive polymer (EAP) actuator disposed in an articulation joint of the shaft is responsive to an electrical signal passed through the shaft to effect articulation. A distal portion of the shaft is pinned to a proximal portion of the shaft forming a pivoting articulation joint. The EAP actuator is connected between the distal and proximal frame portions to effect articulation. Thereby a shaft of an advantageously small diameter may be achieved yet have the functionality of remotely controllable actuation.

In an embodiment of the invention, a surgical instrument has an elongate shaft having a frame assembly and an encompassing and a longitudinally, slidingly received closure sleeve assembly. A staple applying assembly includes an elongate channel, a staple cartridge engaged in the elongate channel, and an anvil pivotally attached to the elongate channel presenting a staple forming surface to the staple cartridge. An articulation joint is formed in the frame assembly. In particular, a distal frame portion is attached to the elongate channel and a proximal frame portion is pivotally pinned to the distal frame portion. A handle attached to a proximal end of the elongate shaft selectively communicates an electrical signal to the elongate shaft to an electroactive polymer actuator connected to the articulation joint that responds thereto to perform articulation of the staple applying assembly. Thus, a surgical stapling and severing instrument is provided that may approach tissue from a desired angle.

These and other objects and advantages of the present invention shall be made apparent from the accompanying drawings and the description thereof.

### Brief Description of the Figures

The accompanying drawings, which are incorporated in and constitute a part of this specification, illustrate embodiments of the invention, and, together with the general description of the invention given above, and the detailed description of the embodiments given below, serve to explain the principles of the present invention.

FIGURE 1 is a rear perspective view of an endoscopic surgical stapling instrument for surgical stapling and severing in an open, unarticulated state.

FIGURE 2 is a perspective view of a laminate Electroactive Polymer (EAP) composite.

FIGURE 3 is a perspective view of an EAP plate actuator formed from a stack formed from an adhesively affixed plurality of laminate EAP composites of FIG. 2.

FIGURE 4 is a perspective view of a cutaway along a longitudinal axis of a contracting EAP fiber actuator.

FIGURE 5 is a front view in elevation taken in cross section along lines 5-5 of the contracting EAP fiber actuator of FIG. 4.

FIGURE 6 is a front right perspective view of an EAP actuated articulation joint for the surgical instrument of FIG. 1 with a flex closure sleeve assembly, a pivoting frame assembly and a closed staple applying assembly.

FIGURE 7 is a front right perspective view of the EAP actuated articulation joint and closed staple applying assembly of FIG. 6 with a flexible closure sleeve assembly removed and a single pivot frame assembly partially exploded.

FIGURE 8 is a front right exploded perspective view of the EAP actuated articulation joint and staple applying assembly of FIG. 6.

FIGURE 9 is a detail view of the exploded single pivot frame assembly including EAP fiber actuators of FIG. 7.

FIGURE 10 is a right side view in elevation taken in cross section along lines 10-10 of FIG. 6 through a pivot axis of the EAP actuated articulation joint and looking right to see a pair of EAP fiber actuators.

FIGURE 11 is top view taken in cross section along lines 11-11 of FIG. 11 through a longitudinal axis of the EAP actuated articulation joint looking down to see a lower moment arm and lower EAP fiber actuators.

FIGURE 12 is a front view in elevation taken in cross section along lines 12-12 of FIG. 10 along the lateral EAP fiber actuators.

FIGURE 13 is a top view of the EAP actuated articulation joint of FIG. 11 with the right upper and lower EAP fiber actuators contracted to articulate the staple applying assembly to the left.

FIGURE 14 is front right perspective view of an additional alternative EAP actuated articulation joint that includes a double pivot closure sleeve assembly in a proximal position opening the anvil of the end effector.

FIGURE 15 is front right exploded view of the additional alternative EAP actuated articulation joint of FIG. 14 including the double pivot closure sleeve assembly and a single pivot frame assembly.

FIGURE 16 is right side view in elevation of the alternative EAP actuated articulation joint taken in cross section along lines 16-16 of FIG. 14 with firing components included.

FIGURE 17 is a top view of the alternative EAP actuated articulation joint in an unarticulated condition taken in cross section along lines 17-17 of FIG. 14.

FIGURE 18 is a top view of the alternative EAP actuated articulation joint in a leftward articulated condition taken in cross section along lines 17-17 of FIG. 14.

FIGURE 19 is yet another alternative EAP actuated articulation joint in a slightly articulated condition with a contracting EAP fiber actuator positioned to straighten the joint.

FIGURE 20 is a right front perspective view of a partially exploded single pivot articulation joint that advantageously includes an EAP articulation locking mechanism that is biased to be normally locked.

FIGURE 21 is a right front perspective view in detail of a proximal portion of the EAP articulation locking mechanism in a proximal frame ground of the single pivot articulation joint.

FIGURE 22 is a top view of the single pivot articulation joint of FIG. 20.

FIGURE 23 is a right side view in elevation of the single pivot articulation joint of FIG. 22 taken in cross section along a longitudinal centerline of lines 23-23.

FIGURE 24 is a top view of the single pivot articulation joint of FIG. 23 taken in cross section along lines 24-24 to show a gear segment on an upper pivot tang locked by the EAP articulation locking mechanism in an unarticulated condition.

FIGURE 25 is a top view of the single pivot articulation joint of FIG. 23 taken in cross section along a centerline of lines 24-24 looking down upon a lower pivot tab of a proximal frame ground that is partially articulating an end effector to the left while the EAP articulation locking mechanism is activated to an unlocked condition.

FIGURE 26 is a front view in elevation of a distal frame ground of the single pivot articulation mechanism of FIG. 24 taken in cross section along lines 26-26 depicting attachment of EAP fiber actuators that articulate the joint.

FIGURE 27 is a front view in elevation of the proximal frame ground of the single pivot articulation joint of FIG. 24 taken in cross section along lines 27-27 to expose EAP stack actuators and locking pins of the EAP actuated locking mechanisms.

FIGURE 28 is a top view taken in cross section along an interface between an upper pivot tang of a distal frame ground and an upper pivot tab of a proximal frame ground of a single pivot articulation joint advantageously incorporating lengthened EAP fiber actuators acting upon rounded moment arms in combination with the EAP articulation locking mechanism.

FIGURE 29 is a front view in elevation taken generally in cross section through the proximal frame ground and EAP articulation locking mechanism but also showing more distally viewed moment arms and lengthened EAP fiber actuators connected thereto.

FIGURE 30 is a top view of a single pivot articulation joint taken in cross section along a top surface of an upper pivot tab of a proximal frame ground to illustrate expansive EAP stack actuators employed against a moment arm distally attached to the upper pivot tab to effect articulation used in conjunction with the normally locked EAP articulation locking mechanism activated in preparation for articulation.

FIGURE 31 is a front view in elevation of the single pivot articulation joint of FIG. 30 taken in cross section through upper and lower tip pins from the moment arms and through the EAP stack actuators.

FIGURE 32 is a top view of the single pivot articulation joint of FIG. 30 taken in cross section along a top surface of the upper pivot tab of the proximal frame ground after articulation of the distal frame ground to the left but before deenergizing the EAP articulation locking mechanism to effect articulation locking.

FIGURE 33 is a front view in elevation of the single pivot articulation joint of FIG. 31 taken in cross section through the upper and lower tip pins from the moment arms and through the expanded left and compressed right EAP stack actuators.

### Detailed Description of the Invention

### Overview Of Articulating Shaft.

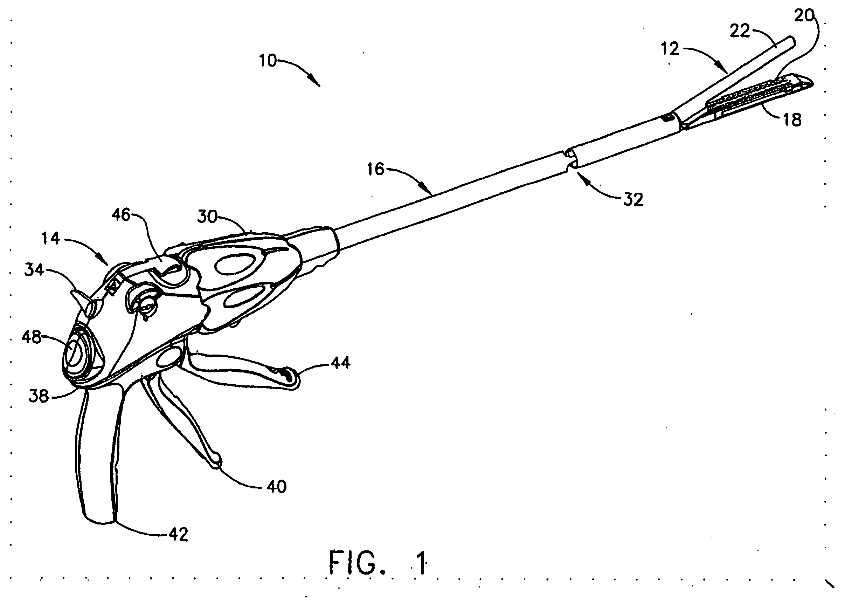

In FIG. 1, a surgical instrument, depicted as a surgical severing and stapling instrument 10, has at its distal end an end effector of a staple applying assembly 12, spaced apart from a handle 14 by an elongate shaft 16. The staple applying assembly 12 includes a staple channel 18 for receiving a replaceable staple cartridge 20. Pivotally attached to the staple channel 18 is an anvil 22 that clamps tissue against the staple cartridge 20 for stapling and severing. When the staple applying assembly 12 is closed, its cross sectional area, as well as the elongate shaft 16 are suitable for insertion through a small surgical opening, such as through a cannula of a trocar (not shown).

Correct placement and orientation of the staple applying assembly 12 is facilitated by controls on the handle 14. In particular, a rotation knob 30 causes rotation of the shaft 16 about its longitudinal axis, and hence rotation of the staple applying assembly 12. Additional positioning is enabled at an articulation joint 32 in the shaft 16 that pivots the staple applying assembly 12 in an arc from the longitudinal axis of the shaft 16, thereby allowing placement behind an organ or allowing other instruments such as an endoscope (not shown) to be oriented behind the staple applying assembly 12. This articulation is advantageously effected by an articulation control switch 34 on the handle 14 that transmits an electrical signal to the articulation joint 32 to an Electroactive Polymer (EAP) actuator 36, powered by an EAP controller and power supply 38 contained within the handle 14.

Once positioned with tissue in the staple applying assembly 12, a surgeon closes the anvil 22 by drawing a closure trigger 40 proximally toward a pistol grip 42. Once clamped thus, the surgeon may grasp a more distally presented firing trigger 44, drawing it back to effect firing of the staple applying assembly 12, which in some applications is achieved in one single firing stroke and in other applications by multiple firing strokes. Firing accomplishes simultaneously stapling of at least two rows of staples while severing the tissue therebetween.

Retraction of the firing components may be automatically initiated upon full travel. Alternatively, a retraction lever 46 may be drawn aft to effect retraction. With the firing components retracted, the staple applying assembly 12 may be unclamped and opened by the surgeon slightly drawing the closure trigger 40 aft toward the pistol grip 42 while depressing a closure release button 48 and then releasing the closure trigger 40, thereby releasing the two stapled ends of severed tissue from the staple applying assembly 12.

It should be appreciated that herein spatial terms such as "vertical", "horizontal", etc. are given with reference to the figures, assuming that the longitudinal axis of the surgical instrument 10 is horizontal with the anvil 22 of the staple applying assembly 12 aligned vertically on top and the triggers 40, 44 aligned vertically on the bottom of the handle 14. However, in actual practice the surgical instrument 10 may be oriented at various angles and as such these spatial terms are used relative to the surgical instrument 10 itself. Further, "proximal" is used to denote a perspective of a clinician who is behind the handle 14 who places the end effector 12 distal, away from himself.

### Handle.

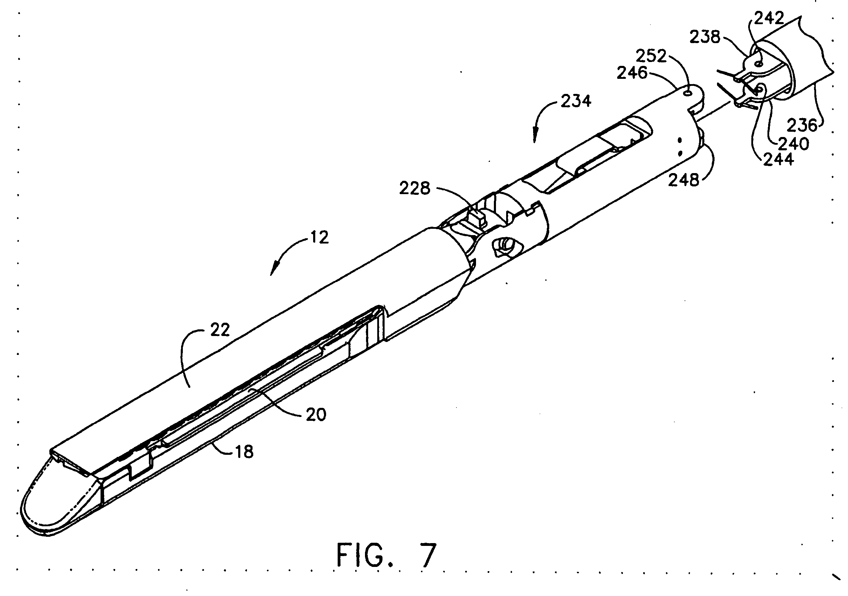

In FIG. 1, the staple applying assembly 12 accomplishes the functions of clamping onto tissue, driving staples and severing tissue by two distinct motions transferred longitudinally down the shaft 16 over a shaft frame (not shown in FIG. 1 but described below regarding FIG. 7). This shaft frame assembly is proximally attached to the handle 14 and coupled for rotation with the rotation knob 30. An illustrative multi-stroke handle 14 for the surgical stapling and severing instrument 10 of FIG. 1 is described in greater detail in the co-pending and co-owned US-A1-2005070958 and US-A1-2005178813.

While a multi-stroke handle 14 advantageously supports applications with high firing forces over a long distance, applications consistent with the present invention may incorporate a single firing stroke, such as described in co-pending and commonly owned US-A1-2005078813.

### Electroactive Polymers.

Electroactive polymers (EAPs) are a set of conductive doped polymers that change shape when an electrical voltage is applied. In essence the conductive polymer is paired to some form of ionic fluid or gel and electrodes. Flow of the ions from the fluid/gel into or out of the conductive polymer is induced by the voltage potential applied and this flow induces the shape change of the polymer. The voltage potential ranges from 1V to 4kV depending on the polymer and ionic fluid used. Some of the EAPs contract when voltage is applied and some expand. The EAPs may be paired to mechanical means such as springs or flexible plates to change the effect that is caused when the voltage is applied.

There are two basic types and multiple configurations of each type. The two basic types are a fiber bundle and a laminate version. The fiber bundle consists of fibers around 30- 50 microns. These fibers may be woven into a bundle much like textiles and are often called EAP yarn because of this. This type of EAP contracts when voltage is applied. The electrodes are usually a central wire core and a conductive outer sheath, which also serves to contain the ionic fluid that surrounds the fiber bundles. An example of a commercially available fiber EAP material is manufactured by Santa Fe Science and Technology and sold as PANION™ fiber and is described in U.S. Pat. No. 6,667,825, which is hereby incorporated by reference in its entirety.

The other type is a laminate structure. It consists of a layer of EAP polymer, a layer of ionic gel and two flexible plates that are attached to either side of the laminate. When a voltage is applied, the square laminate plate expands in one direction and contracts in the perpendicular direction. Commercially available laminate (plate) EAP material is available from Artificial Muscle Inc, a division of SRI Laboratories. Plate EAP material is also available from EAMEX of Japan and is referred to as thin film EAP.

It should be noted that EAPs do not change volume when energized; they merely expand or contract in one direction while doing the opposite in the transverse direction. The laminate version may be used in its basic form by containing one side against a rigid structure and using the other much like a piston. It may also be adhered to either side of a flexible plate. When one side of the flexible plate EAP is energized, it expands, flexing the plate in the opposite direction. This allows the plate to be flexed in either direction depending on which side is energized.

An EAP actuator is usually numerous layers or fibers bundled together to work in cooperation. The mechanical configuration of the EAP determines the EAP actuator and its capabilities for motion. The EAP may be formed into long stands and wrapped around a single central electrode. A flexible exterior outer sleeve will form the other electrode for the actuator as well as contain the ionic fluid necessary for the function of the device. In this configuration when the electrical filed is applied to the electrodes, the strands of EAP shorten. This configuration of EAP actuator is called a fiber EAP actuator. Likewise, the laminate configuration may be placed in numerous layers on either side of a flexible plate or merely in layers on itself to increase its capabilities. Typical fiber structures have an effective strain of 2-4% where the typical laminate version achieves 20-30% utilizing much higher voltages.

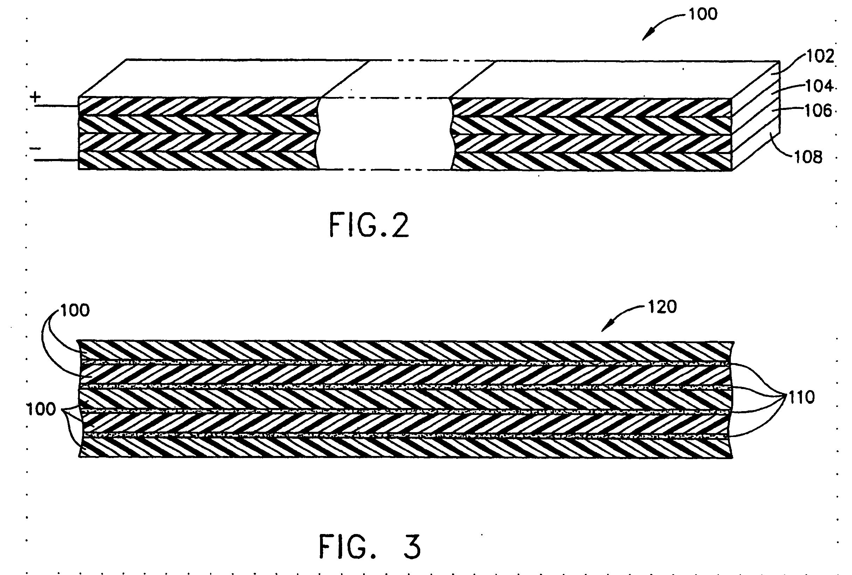

In FIG. 2, a laminate EAP composite 100 is depicted as being formed from a positive plate electrode layer 1302 attached to an EAP layer 104, which in turn is attached to an ionic cell layer 106, which in turn is attached to a negative plate electrode layer 108. In FIG. 3, a plurality of five laminate EAP composites 100 are affixed in a stack by adhesive layers 110 therebetween to form an EAP plate actuator 120. It should be appreciated that opposing EAP actuators 120 may be formed that can selectively bend in either direction.

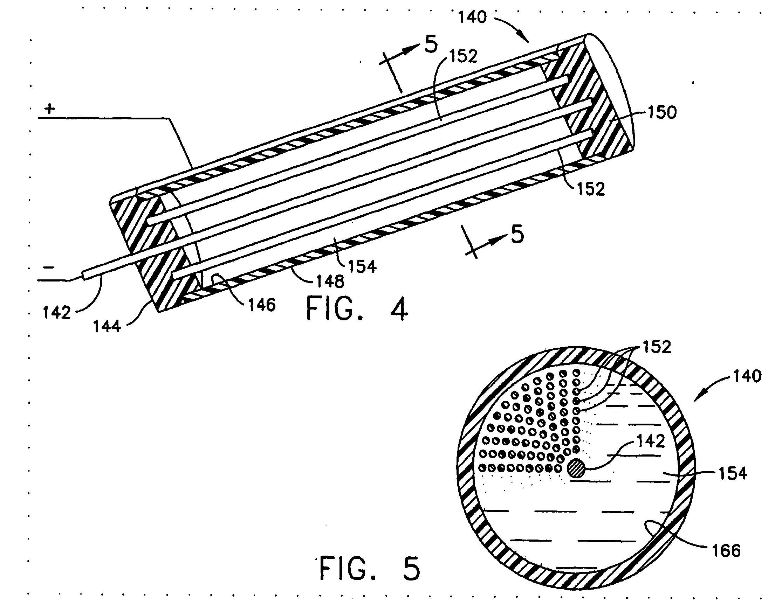

In FIGS. 4-5, a contracting EAP fiber actuator 140 includes a longitudinal platinum cathode wire 142 that passes through an insulative polymer proximal end cap 144 through an elongate cylindrical cavity 146 formed within a plastic cylinder wall 148 that is conductively doped to serve as a positive anode. A distal end of the platinum cathode wire 142 is embedded into an insulative polymer distal end cap 150. A plurality of contracting polymer fibers 152 are arranged parallel with and surrounding the cathode wire 142 and have their ends embedded in respective end caps 144, 150. The plastic cylinder wall 148 is peripherally attached around respective end caps 144, 150 to enclose the cylindrical cavity 146 to seal in ionic fluid or gel 154 that fills the space between contracting polymer fibers 152 and cathode wire 142. When a voltage is applied across the plastic cylinder wall (anode) 148 and cathode wire 142, ionic fluid enters the contracting polymer fibers 152, causing their outer diameter to swell with a corresponding contraction in length, thereby drawing the end caps 144, 150 toward one another. EAP Actuated Articulation Joint.

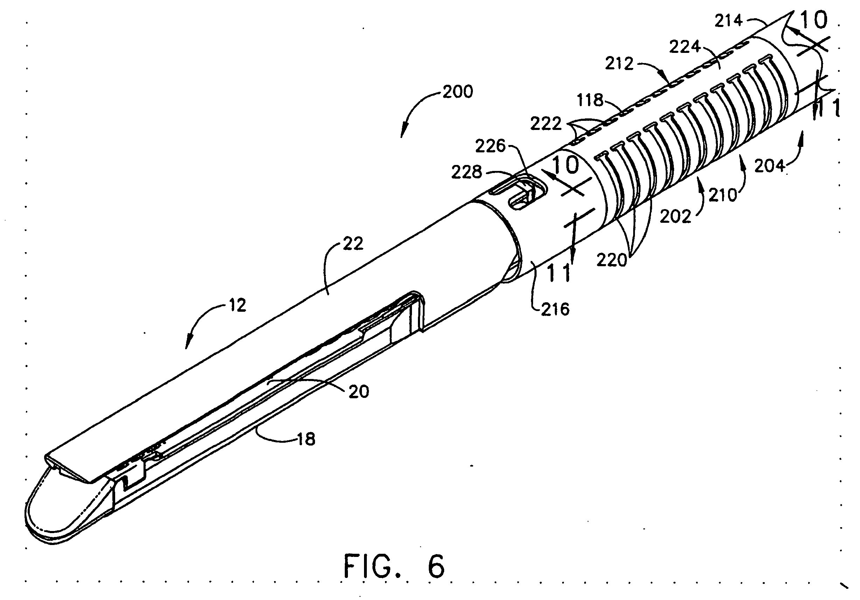

In FIGS. 6-13, a surgical severing and stapling instrument 200 includes an EAP actuated articulation joint 202 that is formed in its elongate shaft 204 proximate to the end effector, which is illustrated by the surgical stapling and severing assembly 12 that advantageously responds to separate closure and firing motions that are transferred longitudinally by the elongate shaft 204. The EAP actuated articulation joint 202 advantageously adds the desirable clinical flexibility of articulating to the staple applying assembly 12.

In the illustrative version of FIGS. 6-13, the EAP actuated articulation joint 202 is more particularly a flexible closure and pivoting frame articulation joint 210, which in FIG. 6 is shown to include a flexible closure sleeve assembly 212 having a proximal closure tube 214 and distal closure ring 216 connected by a flexible closure tube 218. Left and right longitudinal rows of vertical slits 220, 222 formed in the flexible closure tube 218 allow flexing to the right or to the left for articulation, yet an uninterrupted top longitudinal band 224 transfers a longitudinal closure motion regardless of the amount of such flexing. It should be appreciated that an identical uninterrupted bottom longitudinal band runs along the bottom of the flexible closure tube 218 (not shown) is opposite to and cooperates with the top longitudinal band 224 in transferring this motion. In particular, a top portion of the distal closure ring 216 includes a horseshoe aperture 226 that engages an anvil closure feature 228 of the anvil 22. In FIG. 7, the anvil 22 includes laterally projecting pivot pins 230 at its proximal end that pivotally engage pivot apertures 232 formed near the proximal end of the elongate channel 18 (FIGS. 7-8). The slightly more distal anvil closure feature 228 thus imparts a closing motion when the flexible closure sleeve assembly 212 moves distally and imparts an opening motion when moving proximally. The flexible closure tube 218 may bend along the length of the left and right longitudinal rows of vertical slits 220, 222, thus accommodating an encompassed single pivot frame assembly 234 of the flexible closure and pivoting frame articulation joint 210 when articulated.

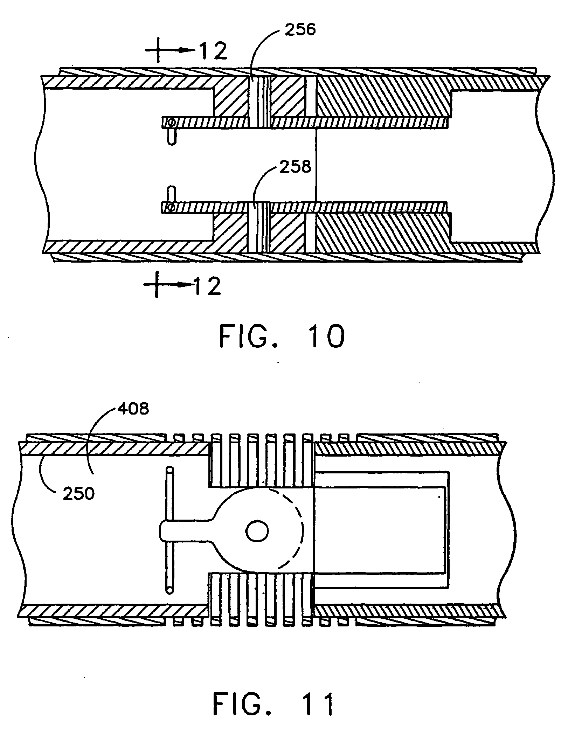

With particular reference to FIGS. 7-9, the single pivot frame assembly 234 includes a proximal frame ground 236 with distally projecting top and bottom pivot tangs 238, 240, each having a respective top and bottom pivot pin hole 242, 244. Corresponding top and bottom pivot tangs 246, 248 projecting proximally from a distal frame ground 250, each tang 246, 248 with respective top and bottom pivot pin holes 252, 254, pivotally engage the proximal frame ground 236. In particular, the vertically aligned top pivot pin holes 242, 252 and bottom pivot pin holes 244, 254 are respectively engaged by top and bottom frame pivot pins 256, 258 (FIG.10).

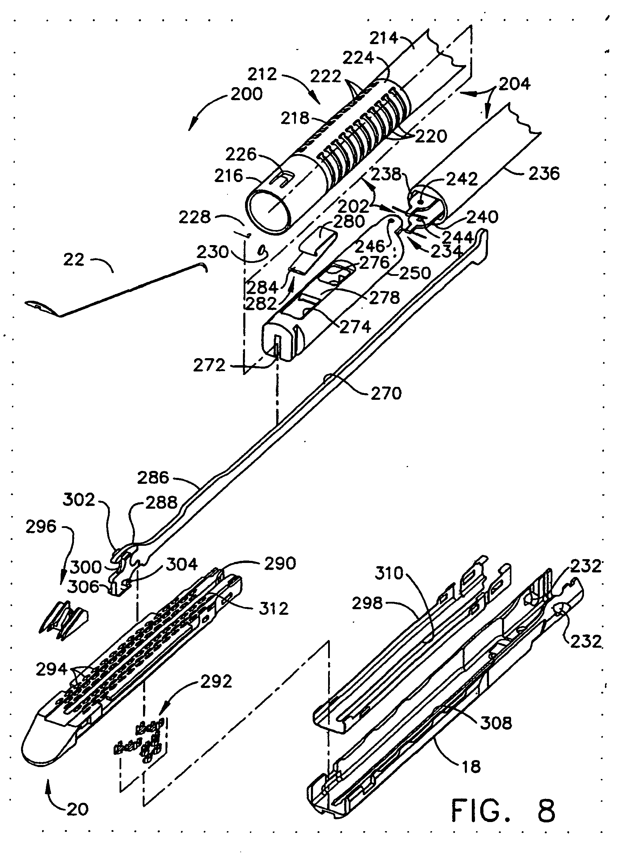

In FIG. 8, an implement portion 260 of the surgical instrument 200, formed by the elongate shaft 16 and staple applying assembly 12, further includes a firing bar 270 that longitudinally translates through the proximal frame ground 218, through the flexible closure and pivoting frame articulation joint 210, and through a firing slot 272 in the distal frame ground 250 into the staple applying assembly 12. Distal and proximal square apertures 274, 276, formed on top of the distal frame ground 250, define a clip bar 278 therebetween that receives a top arm 280 of a clip spring 282 whose lower, distally extended arm 284 asserts a downward pressure on a raised portion 286 along an upper portion of the firing bar 270 corresponding to the empty/missing cartridge lockout portion of firing travel.

With particular reference to FIG. 8, a distally projecting end of the firing bar 270 is attached to an E-beam 288 that assists in spacing the anvil 22 from the staple cartridge 20, severs tissue, and actuates the staple cartridge 20. The staple cartridge 20 includes a molded cartridge body 290 that holds a plurality of staples resting upon staple drivers 292 within respective upwardly open staple apertures 294. A wedge sled 296 is driven distally by the E-beam 28 21'8, sliding upon a cartridge tray 298 that holds together the various components of the replaceable staple cartridge 20. The wedge sled 296 upwardly cams the staple drivers 292 to force out the staples into deforming contact with the anvil 22 while a cutting surface 300 of the E-beam 288 severs clamped tissue. It should be appreciated that upper pins 302 of the E-beam 288 engage the anvil 22 during firing while middle pins 304 and a bottom foot 306 engage respective top and bottom surfaces into a longitudinal slot 308 formed in the elongate channel 18, with a corresponding longitudinal opening 310 in the cartridge tray 298 and a rearwardly open vertical slot 312 in the cartridge body 290. Thereafter, the firing bar 270 is retracted proximally, retracting as well the E-beam 288, allowing the anvil 22 to be opened to release the two stapled and severed tissue portions (not shown).

The staple applying assembly 12 is described in greater detail in co-pending and commonly-owned US-A1-2005263562.

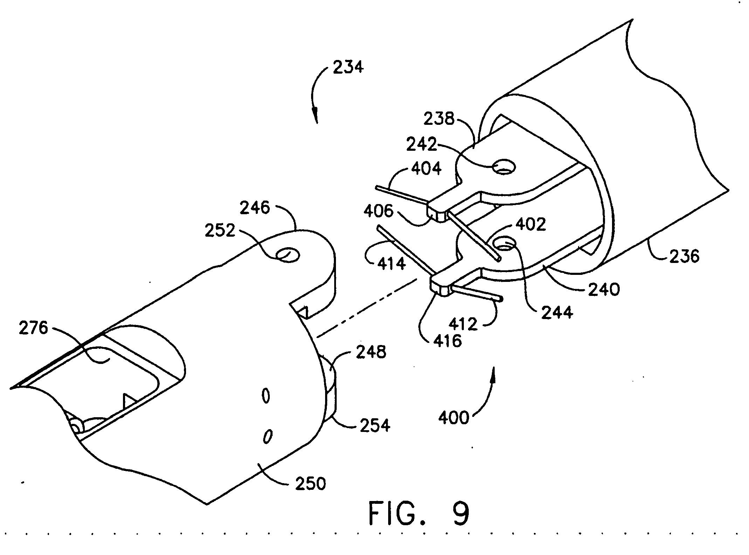

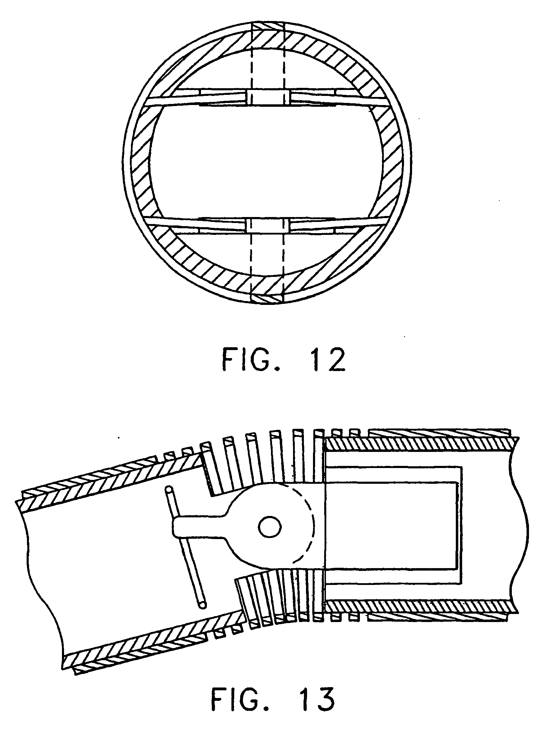

With particular reference to FIGS. 9-13, an EAP actuator system 400 advantageously actuates the single pivot frame assembly 234 in response to an electrical articulation signal (not shown) received from the handle 14. In the illustrative version of FIGS. 7-13, top left and top right EAP fiber actuators 402, 404 attach horizontally to each lateral side of a top distally projecting moment arm 406 attached to the top pivot tang 238. The outer ends of the top left and top right EAP fiber actuators 402, 404 are attached to respective upper left and right lateral attachment points 406, 408 of an inner diameter 410 of the distal frame ground 250. Similar, bottom left and bottom right EAP fiber actuators 412, 414 attach horizontally to each lateral side of a bottom distally projecting moment arm 416 attached to the bottom pivot tang 240. The outer ends of the bottom left and bottom right EAP fiber actuators 412, 414 are attached to respective lower left and right lateral attachment points 418, 420 of the inner diameter 410 of the distal frame ground 250. The attachment points 406, 408, 418, 420 are shown to pass through the distal frame ground 250 in FIG. 12 with the left attachment points 406, 418 visible on the exterior of the distal frame ground 250 in FIG. 9. When activating one pair of EAP actuators, such as in FIG. 13, the upper and lower right EAP fiber actuators 404, 414 cause them to contract, drawing the upper and lower moment arms 406, 416 toward the right side of the distal frame ground 250, thereby stretching the upper and lower EAP fiber actuators 402, 412, collapsing the left longitudinal row of vertical slits 220, and expanding the right longitudinal row of vertical slits 222.

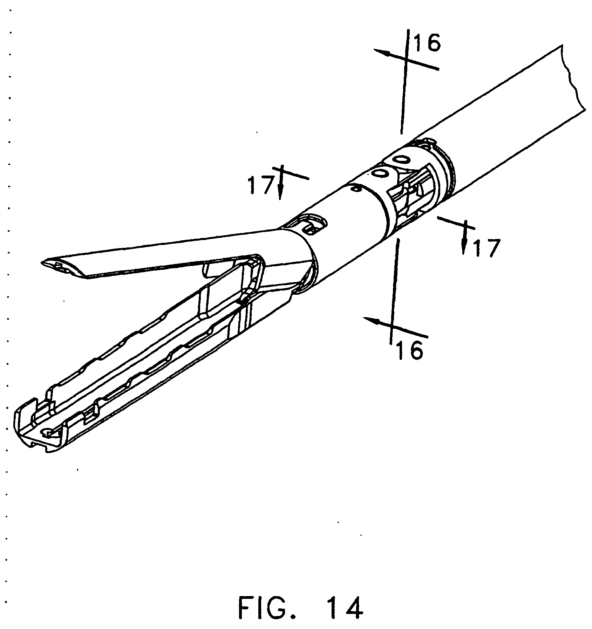

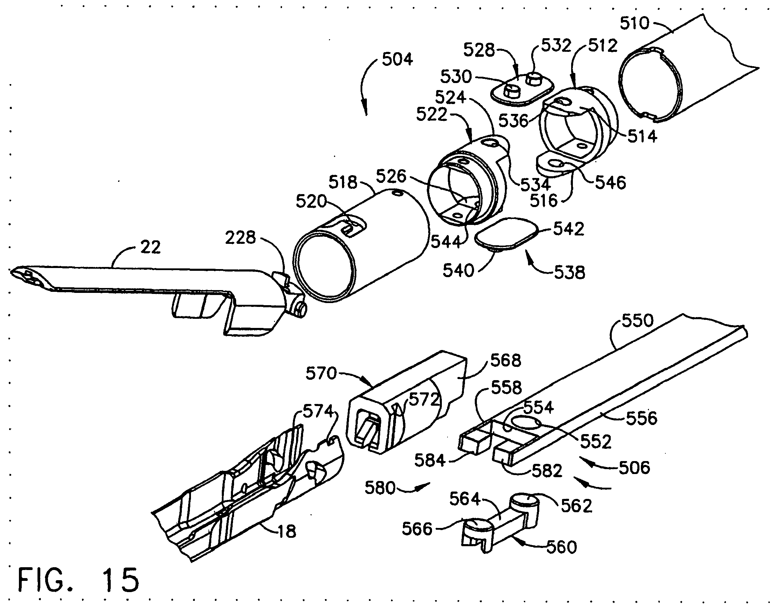

In FIGS. 14-18, a surgical severing and stapling instrument 500 includes an alternative EAP actuated articulation joint 502 that includes a double pivot closure sleeve assembly 504 (FIG. 14-15) and a single pivot frame assembly 506 (FIG. 15-18). In FIG. 14, the staple applying assembly 12 is depicted with the replaceable staple cartridge 20 removed and the anvil 22 open. Thus, the double pivot closure sleeve assembly 504 is at its proximal position with its distal pivoting axis aligned with a pivoting axis of the frame assembly 506. It should be appreciated that with the closure sleeve assembly 504 moved distally to close the anvil 22, a proximal pivot axis of the closure sleeve assembly 504 also pivots in order to translate over an articulated frame assembly 506.

With particular reference to FIG. 15, the double pivot closure sleeve assembly 504 includes a proximal closure tube 510 whose distal end is keyed to attach to a proximal closure ring 512 having upper and lower distally projecting tangs 514, 516. A distal closure tube 518, which includes a horseshoe aperture 520 to engage the anvil closure feature 228 on the anvil 22, is proximally pinned to a distal closure ring 522 having upper and lower proximally projecting tangs 524, 526. An upper double pivot link 528 includes upwardly projecting distal and proximal pivot pins 530, 532 that engage respectively an upper distal pin hole 534 in the upper proximally projecting tang 524 and an upper proximal pin hole 536 in the upper distally projecting tang 514. A lower double pivot link 538 includes downwardly projecting distal and proximal pivot pins 540, 542 that engage respectively a lower distal pin hole 544 in the lower proximally projecting tang 526 and a lower proximal pin hole 546 in the lower distally projecting tang 516.

With particular reference to FIGS. 15-18, the single pivot frame assembly 506 includes a proximal frame ground 550 whose distal end includes a pivot pin hole 552 centered and proximal to a distally open pivot recess 554 defined between left and right moment arms 556, 558. A dog bone link 560 includes a proximal pin 562 that upwardly engages the pivot pin hole 552 in the proximal frame ground 550 and a center bar 564 that pivots between the left and right moment arms 556, 558. A distal pin 566 of the dog bone link 560 is rigidly attached into a lower proximal bore 568 in a distal frame ground 570 having distal lateral guides 572 that engage proximal guides 574 in the elongate channel 18.

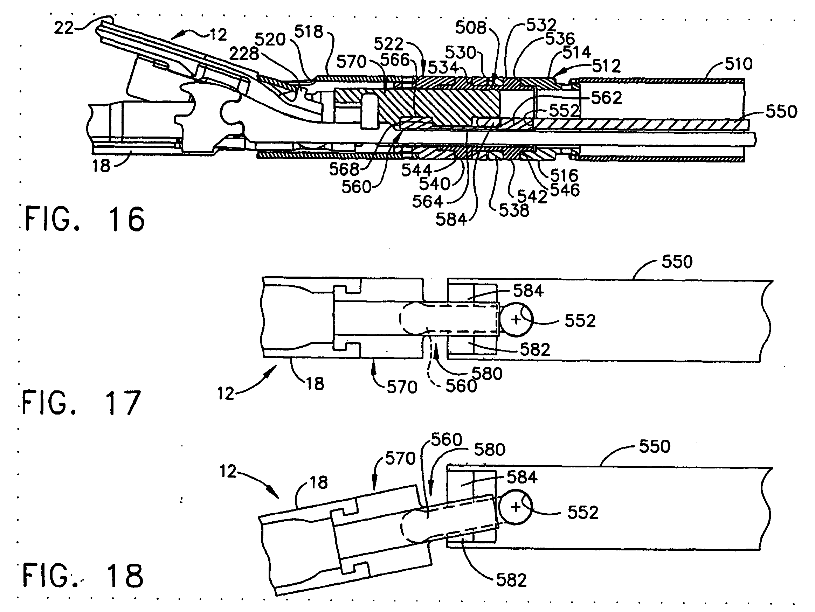

An EAP actuation system 580 includes left and right EAP stack actuators 582, 584 that selectively expand to assert an articulation force on the center bar 564 of the dog bone link 560, which passively compresses the other EAP stack actuator. In FIG. 18, the right EAP stack actuator 582 has expanded, pivoting the dog bone link 560 and thus the staple applying assembly 12 to the left and passively compressing the left EAP stack actuator 584.

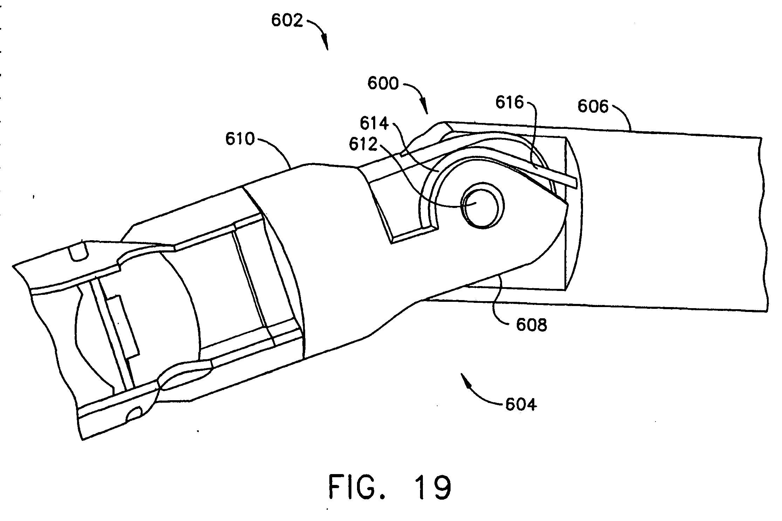

In FIG. 19, yet another alternative EAP actuated articulation joint 600 for a surgical instrument 602 includes a single pivoting frame assembly 604 wherein a proximal frame ground 606 is engaged to a distally projecting tang 608 from a distal frame ground 610 at a pivot pin 612. The distally projecting tang 608 is recessed on a right lateral side to define a half teardrop shaped pulley 614 on the right side of the pivot pin 612. Attached to a distal point of the half teardrop shaped pulley 614 is a distal end of a contracting EAP fiber actuator 616 that follows the contour thereof and passes into the proximal frame ground 606. The contracting EAP fiber actuator 616 may be sufficiently long so that for even a small percentage contraction in a length a significant rotation may be achieved. It should be appreciated that a counter rotating mechanism may be incorporated on a left side of the depicted tang 608 on a similar but reversed mechanism formed on the other side of the EAP articulation joint 600. Articulation Locking Mechanism For Pivoting Articulation Mechanism.

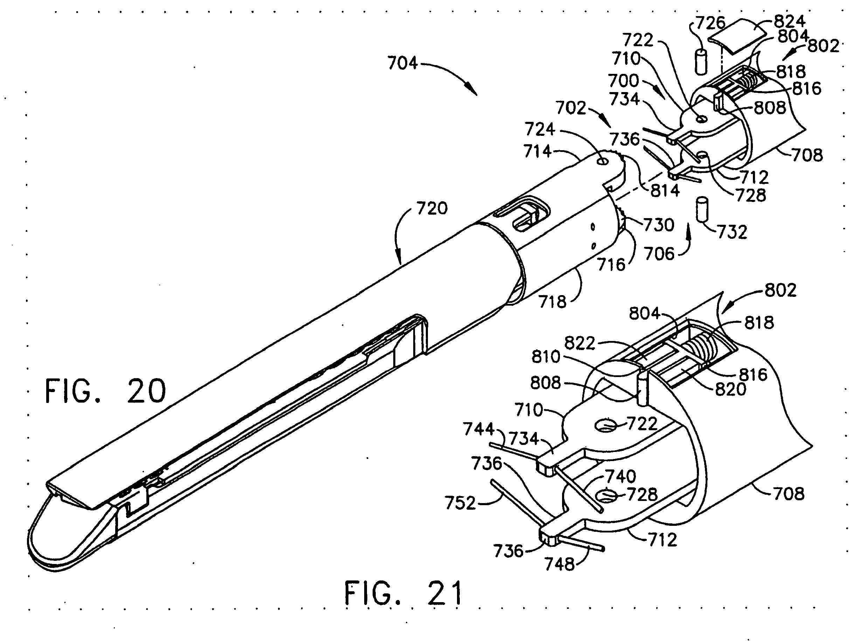

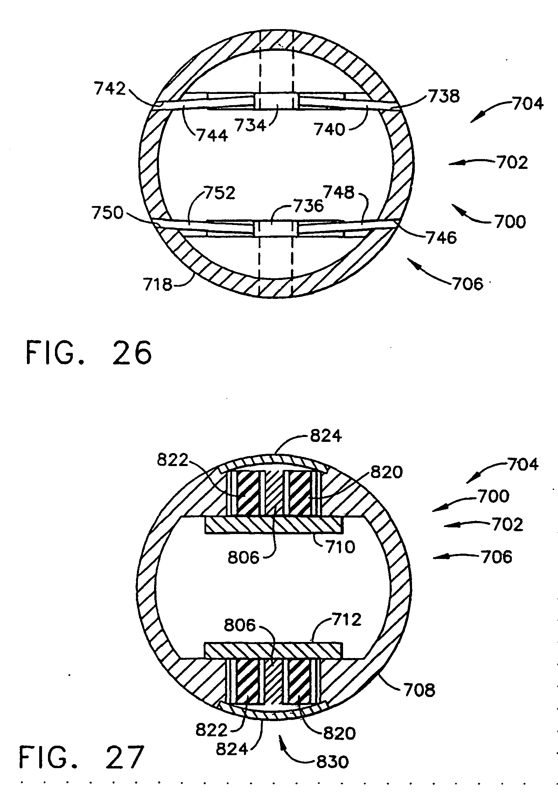

In FIGS. 20-27, an EAP actuated articulation lock 700 is incorporated into a pivoting articulation joint 702 for a surgical instrument 704. For clarity, a single pivoting frame assembly 706 is depicted with a proximal frame ground 708 having distally extended upper and lower pivot tabs 710, 712 that are pivotally engaged to proximally directed upper and lower tangs 714, 716 of a distal frame ground 718 that is attached to an end effector 720. An upper inner hole 722 in the upper pivot tab 710 is aligned under an upper outer hole 724 in the upper tang 714, which are pivotally pinned together by upper pivot pin 726. A lower inner hole 728 in the lower pivot tab 712 is aligned above a lower outer hole 730 in the lower tang 716, which are pivotally pinned together by a lower pivot pin 732. Upper and lower moment arms 734, 736 extend distally respectfully from the upper and lower pivot tabs 710, 712. The upper moment arm 734 may be urged to the left toward an upper left attachment point 738 formed in the distal frame ground 718 by a generally horizontal upper left EAP fiber actuator 740. The upper moment arm 734 may be urged to the right toward an upper right attachment point 742 formed in the distal frame ground 718 by a generally horizontal upper right EAP fiber actuator 744. The lower moment arm 736 may be urged to the left toward a lower left attachment point 746 formed in the distal frame ground 718 by a generally horizontal lower left EAP fiber actuator 748. The lower moment arm 736 may be urged to the right toward a lower right attachment point 750 formed in the distal frame ground 718 by a generally horizontal lower right EAP fiber actuator 752.

Closure of the anvil 22 may occur by action of a closure mechanism that is not shown, such as an EAP actuator that acts upon the anvil pivot. Alternatively, a firing motion may first close the anvil prior to further motion effecting stapling and severing. As a further alternative, a closure sleeve assembly or other longitudinally coupled mechanism (not shown) may impart a closing motion to the anvil 22.

An upper EAP actuated articulation locking mechanism 800 advantageously unlocks the pivoting articulation joint 702 to allow articulating movement. The EAP actuated articulation locking mechanism 800 then relaxes to a locked state, providing a stable locked position that does not require power dissipation, and thus component heating, between changes in an amount of articulation. An upper locking bolt assembly 802 is shown in a rectangular upper lock recess 804 formed in the proximal frame ground 708 proximal to and vertically farther from the longitudinal centerline than the upper pivoting tab 710. A locking bolt 806 extends a locking tip 808 out of a distal slot 810 formed in the upper lock recess 804 into engagement in a nearest tooth root 812 of a gear segment 814 formed about a proximal surface about the upper pivot tang 714 of the distal frame ground 718. The locking bolt 806 proximally terminates in a cross plate 816 that slides longitudinally in the rectangular upper lock recess 804 between the urging of a proximally positioned compression spring 818 and upper left and right EAP stack actuator 820, 822 that may be activated to expand longitudinally, compressing the compression spring 818 as the lock bolt 806 is moved proximally, thereby disengaging the locking tip 808 from the gear segment 814, allow the pivoting articulation joint 702 to be repositioned. An upper lock cover 824 closes the upper lock recess 804.

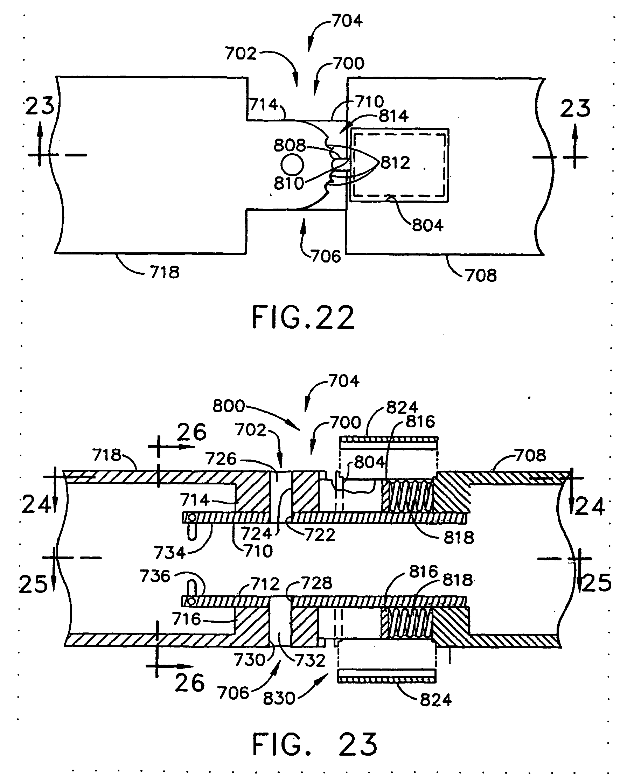

For additional locking support, in FIG. 23, a lower EAP actuated articulation locking mechanism 830 is identical to the upper locking mechanism 800 but acting on the opposite site against lower pivot tang 716. It should further be appreciated that a similar locking mechanism may be incorporated into a distal portion of an elongate shaft rather than a proximal end. Further, a double pivoting coupling may include a lock at each pivot.

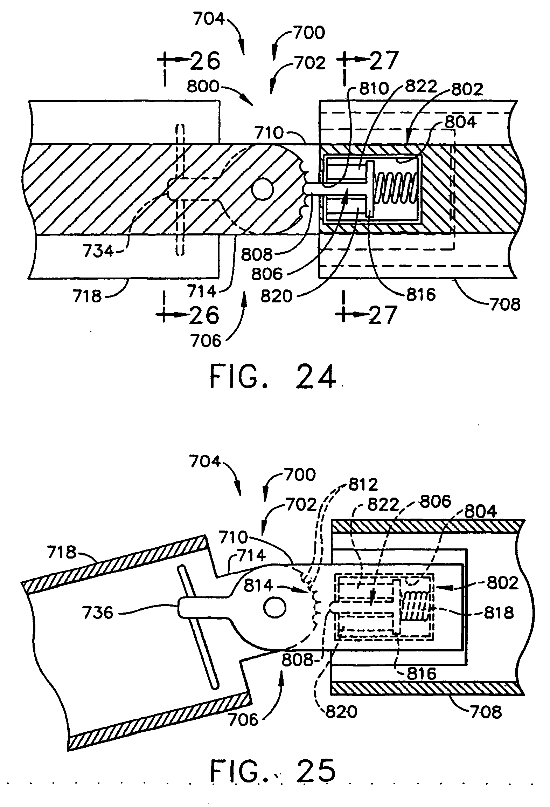

In use, an unarticulated end effector 720 and pivoting articulation joint 702 (FIGS. 20-24) is inserted into a surgical site. With EAP locking mechanisms 800, 830 typically deenergized, the locking tip 808 attached to the proximal frame ground 708 engages the gear segment 814 of the distal frame ground 718, locking the single pivot frame assembly 706. When desired, EAP stack actuators 820, 820 are energized to longitudinally lengthen, unlocking the EAP articulation locking mechanisms 800, 830. While unlocked, the articulation joint 702 may be articulated, such as by contracting upper and lower right EAP fiber actuators 744, 752 to pivot the end effector 720 to the left (FIG. 25), presenting a different tooth root 812 to the locking tip 808 so that when deenergized the EAP articulation locking mechanism 800 will lock to the articulation condition of the surgical instrument 704.

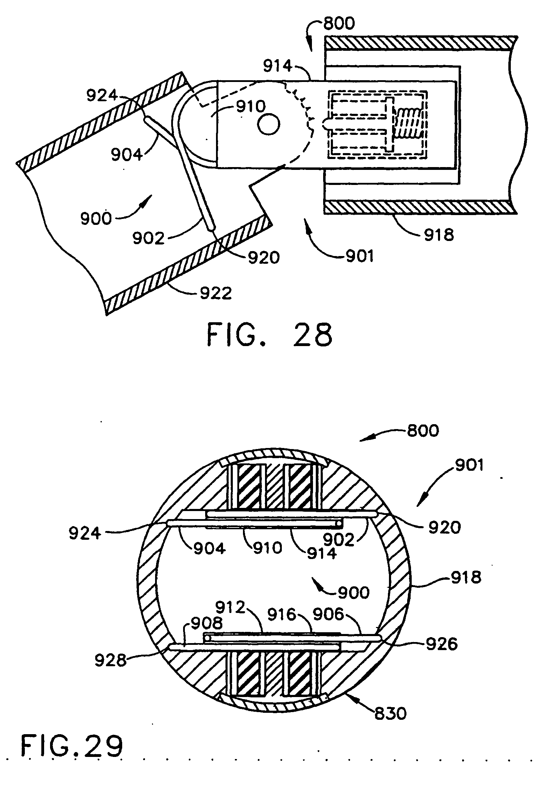

In FIGS. 28-29, an alternative EAP articulation system 900 for a single pivot articulation joint 901 is depicted for use in conjunction with the EAP articulation locking mechanism 800 previously described. Upper and lower pairs of left and right EAP fiber actuators 902, 904, 906, 908 are lengthened by incorporating upper and lower rounded moment arms 910, 912 distally respectively on upper and lower pivot tabs 914, 916 of a proximal frame ground 918. An upper left attachment point 920 in a distal frame ground 922 is slightly higher than an upper right attachment point 924 and a lower left attachment point 926 is also slightly higher than a lower right attachment point 928, corresponding to the upper and lower left EAP fiber actuators 902, 906 wrapping respectively around a higher portion of the corresponding upper and lower rounded moment arms 910, 912 than the upper and lower right EAP fiber actuators 904, 908 (FIG. 29). Thereby, the lengthened EAP fiber actuators 902-908 in combination with the length and contour of the moment arms 910, 912 may be selected as a desirable performance characteristic.

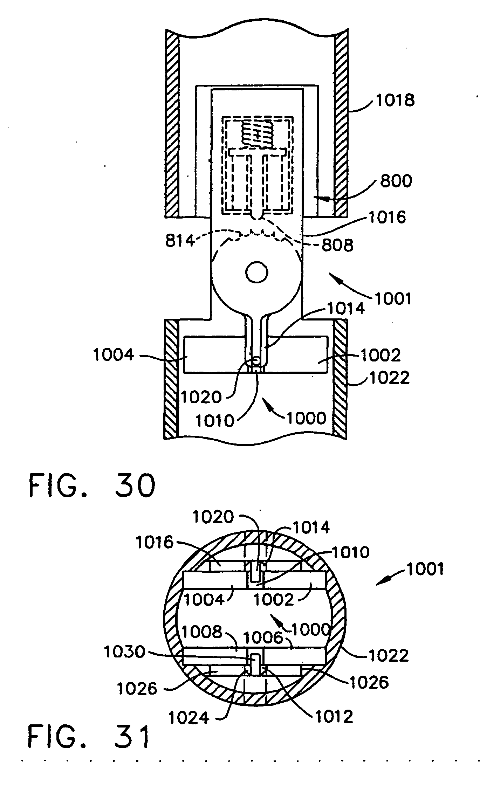

In FIGS. 30-33, an additional alternative EAP articulation system 1000 for a single pivot articulation joint 1001 is depicted for use in conjunction with the EAP articulation locking mechanism 800 previously described. Instead of EAP fiber actuators that effect articulation, upper and lower pairs of left and right EAP stack actuators 1002, 1004, 1006, 1008 respectively oppose and laterally move upper and lower longitudinal tracks 1010, 1012. A distally projecting upper moment arm 1014 attaches to an upper pivot tab 1016 of a proximal frame ground 1018. An upper inwardly directed tip pin 1020 at a distal end of the upper moment arm 1014 longitudinally slidingly engages the upper longitudinal track 1010, and thus responds to the differential contraction and expansion of the upper left and right EAP stack actuators 1002, 1004 that are laterally constrained by a distal frame ground 1022. A distally projecting lower moment arm 1024 attaches to an upper pivot tab 1026 of the proximal frame ground 1018. A lower inwardly directed tip pin 1030 at a distal end of the upper moment arm 1024 longitudinally slidingly engages the lower longitudinal track 1012, and thus responds to the differential contraction and expansion of the lower left and right EAP stack actuators 1006, 1008 that are laterally constrained by the distal frame ground 1022.

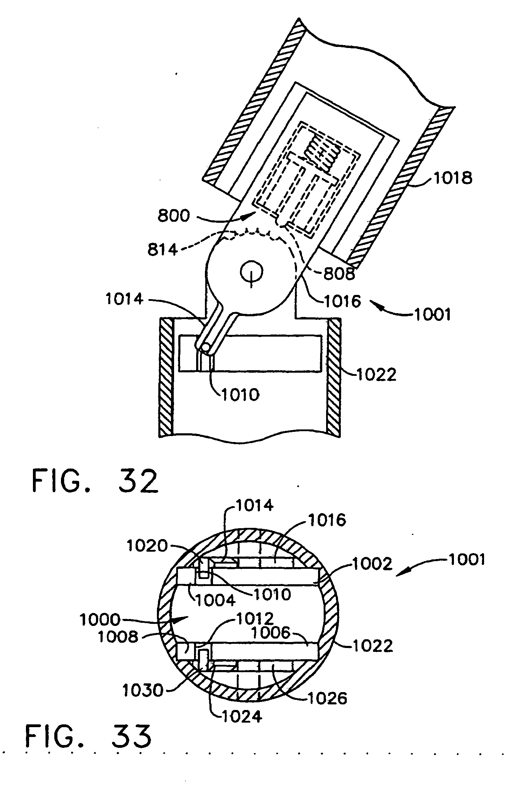

In FIGS. 30-31, the EAP articulation locking mechanism 800 is activated to disengage the locking tip 808 from the gear segment 814 in preparation for articulation. In FIGS. 32-33, the upper and lower left EAP stack actuators 1002, 1006 have been energized to expand, laterally moving rightward the upper and lower longitudinal tracks 1010, 1012, thereby compressing the upper and lower EAP stack actuators 1004, 1008 and moving distal frame ground 1022 correspondingly against the reaction force from the upper and lower inwardly directed tip pins 1020, 1030, which in the illustrative articulation is to the left.

## Claims

1. A surgical instrument (200), comprising:
an end effector (12);
an elongate shaft (204);
a pivoting articulation joint (202) including a first frame member (250) attached to a selected one of the end effector (12) and a distal end of the elongate shaft (204), a second frame member (236) attached to the other one of the end effector (12) and the distal end of the elongate shaft (204), and a pivoting connection between the first and second frame members (250, 236); and an electroactive polymer actuator (400) connected between the first and second frame members (250, 236);
**characterised in that** the first frame member (250) includes a recess opening toward the pivoting connection and the second frame member (236), the second frame member (236) including a first moment arm (406, 416) extending into the recess, the actuator (400) being attached between the moment arm (406, 416) and the first frame member (250) across the recess.

2. The surgical instrument (200) of claim 1, wherein the electroactive polymer actuator (400) comprises an electroactive polymer fiber actuator (402, 404, 412, 414) operatively configured to contract.

3. The surgical instrument (200) of claim 1, wherein the first frame member (250) includes a first upper tang (246) and a lower first tang (248) pivotally attached respectively to an upper second tang (238) and a lower second tang (240) of the second frame member (236), the upper first and second tangs (246, 238) being laterally spaced from the lower first and second tangs (248, 240).

4. The surgical instrument (200) of claim 3, wherein the first frame member (250) comprises a tube defining a recess that receives the second frame member (236), at least one of the upper and lower second tangs (238, 240) including a moment arm (406, 416) extending into the recess, the electroactive polymer actuator (400) comprising a pair of opposing electroactive polymer actuators (402, 404, 412, 414) attached to the moment arm (406, 416) and respective lateral interior surfaces (406, 408, 418, 420) of the first frame member (250).

5. The surgical instrument (602) of claim 3, wherein at least one tang (608) includes a circumferentially contoured portion (614) transverse to an axis of articulation of the articulation joint (600), the electroactive polymer actuator (400) comprising an electroactive polymer fiber actuator (616) attached to and positioned upon the circumferentially contoured portion (614) at one end and attached to the other frame member (606).

6. The surgical instrument (602) of claim 5, wherein at least one tang (608) includes a counter circumferentially contoured portion transverse to the axis of articulation of the articulation joint (600), the surgical instrument (602) further comprising a counter electroactive polymer fiber actuator attached to and positioned on the counter circumferential portion.

7. The surgical instrument (602) of claim 6, wherein a selected one of the upper tangs (246, 238) and a selected one of the lower tangs (248, 240) both include a rounded contour including an upper portion attached to the electroactive polymer fiber (616) and including a lower portion attached to the counter electroactive fiber actuator.

8. The surgical instrument (200) of claim 3, wherein the end effector (12) comprises a stapling and severing assembly (12) actuated by a firing bar (270) and a handle portion (14) proximally attached to the firing bar (270) and operably configured to impart longitudinal firing motion to the firing bar (270), the elongate shaft (204) further comprising a firing bar guide (272) supporting the firing bar (270) through an articulated articulation joint (202).

9. The surgical instrument (200) of claim 8, wherein the end effector (12) further comprises a lower channel (18) operatively configured to receive a staple cartridge (20) and comprises a pivotally attached upper anvil (22) and a handle portion (14) operatively configured to produce a longitudinal closure motion, the elongate shaft (204) further comprising a closure sleeve assembly (228) proximally coupled to the handle portion (14) to transfer the closure motion to a distal connection with the anvil (22), the closure sleeve assembly (228) operatively configured to pivot about an axis of articulation of the articulation joint (202) in both a retracted position and distally extended position.

10. The surgical instrument (704) of claim 1, further comprising an articulation locking mechanism (800) comprising:
a longitudinally translating locking member (806) attached to a selected one of the first and second frame member (718, 708) and biased to extend toward and engage the other one of the first and second frame member (718, 708); and
an electroactive polymer actuator (820, 822) operatively configured and positioned to overcome the bias on the longitudinally translating locking member (806) when activated to unlock the articulation joint (702).

11. The surgical instrument (200) of claim 1, wherein
the elongate shaft (204) comprises a frame assembly (210) encompassed by a longitudinally, slidingly received closure sleeve assembly (212);
the end effector (12) comprises a staple applying assembly (12) comprising an elongate channel (18), a staple cartridge (20) engaged in the elongate channel (18), and an anvil (22) pivotally attached to the elongate channel (18) presenting a staple forming surface to the staple cartridge (20);
the electroactive polymer actuator (400) performs articulation of the staple applying assembly (12);
and further comprising a handle portion (14) attached to a proximal end of the elongate shaft (16) and operatively configured to selectively communicate an electrical signal to the elongate shaft (16).

## Patentansprüche

1. Chirurgisches Instrument (200), umfassend:
einen Endeffektor (12);
einen länglichen Schaft (204);
eine Dreh-Gelenkverbindung (202), einschließlich eines ersten Rahmenelements (250), befestigt entweder an dem Endeffektor (12) oder an einem distalen Ende des länglichen Schafts (204), ein zweites Rahmenelement (236), befestigt an dem anderen des Endeffektors (12) und des distalen Endes des länglichen Schafts (204), und eine Drehverbindung zwischen dem ersten und dem zweiten Rahmenelement (250, 236); und
einen elektroaktiven Polymerbetätiger (400), verbunden zwischen dem ersten und dem zweiten Rahmenelement (250, 236),
**dadurch gekennzeichnet, dass** das erste Rahmenelement (250) eine Vertiefungsöffnung in Richtung auf die Drehverbindung und das zweite Rahmenelement (236) einschließt, das zweite Rahmenelement (236) einen ersten Hebel (406, 416) einschließt, der sich in die Vertiefung hinein erstreckt, wobei der Betätiger (400) zwischen dem Hebel (406, 416) und dem ersten Rahmenelement (250) über die Vertiefung hinweg befestigt ist.

2. Chirurgisches Instrument (200) nach Anspruch 1, **dadurch gekennzeichnet, dass** der elektroaktive Polymerbetätiger (400) einen elektroaktiven Polymerfaserbetätiger (402, 404, 412, 414) umfasst, der dafür ausgelegt ist, sich bei der Betätigung zusammenzuziehen.

3. Chirurgisches Instrument (200) nach Anspruch 1, **dadurch gekennzeichnet, dass** das erste Rahmenelement (250) einen oberen ersten Zapfen (246) und einen unteren ersten Zapfen (248) einschließt, schwenkbar befestigt jeweils an einem oberen zweiten Zapfen (238) und einem unteren zweiten Zapfen (240) des zweiten Rahmenelements (236), wobei der obere erste Zapfen (246) und der obere zweite Zapfen (238) seitlich vom unteren ersten Zapfen (248) und vom unteren zweiten Zapfen (240) beabstandet sind.

4. Chirurgisches Instrument (200) nach Anspruch 3, **dadurch gekennzeichnet, dass** das erste Rahmenelement (250) ein Rohr umfasst, welches eine Vertiefung begrenzt, die das zweite Rahmenelement (236), zumindest einen des oberen und des unteren zweiten Zapfens (238, 240) aufnimmt, einschließlich eines Hebel (406, 416), der sich in die Vertiefung hinein erstreckt, wobei der elektroaktive Polymerbetätiger (400) ein Paar einander gegenüberliegender elektroaktiver Polymerbetätiger (402, 404, 412, 414) umfasst, die am Hebel (406, 416) und den jeweiligen seitlichen inneren Oberflächen (406, 408, 418, 420) des ersten Rahmenelements (250) befestigt sind.

5. Chirurgisches Instrument (602) nach Anspruch 3, **dadurch gekennzeichnet, dass** zumindest ein Zapfen (608) einen umfänglich konturierten Abschnitt (614) quer zu einer Achse des Gelenks der Gelenkverbindung (600) einschließt, und der elektroaktive Polymerbetätiger (400) einen elektroaktiven Polymerfaserbetätiger (616) umfasst, der an dem umfänglich konturierten Abschnitt (614) an einem Ende befestigt ist und auf diesem positioniert ist und am anderen Rahmenelement (606) befestigt ist.

6. Chirurgisches Instrument (602) nach Anspruch 5, **dadurch gekennzeichnet, dass** zumindest ein Zapfen (608) einen umfänglich konturierten Gegen-Abschnitt quer zur Achse des Gelenks der Gelenkverbindung (600) einschließt, wobei das chirurgische Instrument (602) weiterhin einen elektroaktiven Gegen-Polymerfaserbetätiger umfasst, der an dem umfänglichen Gegen-Abschnitt befestigt ist und auf diesem positioniert ist.

7. Chirurgisches Instrument (602) nach Anspruch 6, **dadurch gekennzeichnet, dass** ein ausgewählter der oberen Zapfen (246, 238) und ein ausgewählter der unteren Zapfen (248, 240) beide eine gerundete Kontur einschließen, einschließlich eines oberen Abschnitts, der an der elektroaktiven Polymerfaser (616) befestigt ist, und einschließlich eines unteren Abschnitts, der am elektroaktiven Gegen-Faserbetätiger befestigt ist.

8. Chirurgisches Instrument (200) nach Anspruch 3, **dadurch gekennzeichnet, dass** der Endeffektor (12) eine Klammer- und Abtrennanordnung (12) umfasst, die durch eine Abfeuerungsstange (270) betätigt wird, und einen Griffabschnitt (14), proximal an der Abfeuerungsstange (270) befestigt, und der betriebsfähig gestaltet ist, um der Abfeuerungsstange (270) eine longitudinale Abfeuerungsbewegung zu verleihen, wobei der längliche Schaft (204) weiterhin eine Abfeuerungsstangenführung (272) umfasst, welche die Abfeuerungsstange (270) mit Hilfe einer Dreh-Gelenkverbindung (202) trägt.

9. Chirurgisches Instrument (200) nach Anspruch 8, **dadurch gekennzeichnet, dass** der Endeffektor (12) weiterhin einen unteren Kanal (18) umfasst, der betriebsfähig gestaltet ist für die Aufnahme einer Klammerkassette (20), und einen schwenkbar befestigten oberen Amboss (22) und einen Griffabschnitt (14) umfasst, betriebsfähig gestaltet für das Erzeugen einer longitudinalen Verschließbewegung, wobei der längliche Schaft (204) weiterhin eine Verschlussmanschettenanordnung (228) umfasst, proximal mit dem Griffabschnitt (14) gekoppelt, um die Verschließbewegung zu einer distalen Verbindung mit dem Amboss (22) zu übertragen, wobei die Verschlussmanschettenanordnung (228) wirksam gestaltet ist für das Schwenken um eine Achse des Gelenks der Gelenkverbindung (202) sowohl in einer zurückgezogenen Position als auch in einer distal ausgefahrenen Position.

10. Chirurgisches Instrument (704) nach Anspruch 1, weiterhin umfassend eine Gelenkarretiereinrichtung (800), umfassend:
ein longitudinal translatierendes Arretierelement (806), befestigt an einem ausgewählten des ersten und des zweiten Rahmenelements (718, 708) und vorgespannt, um sich hin zu dem anderen des ersten und des zweiten Rahmenelements (718, 708) zu erstrecken und diese in Eingriff zu nehmen; und
einen elektroaktiven Polymerbetätiger (820, 822),'wirksam gestaltet und positioniert, um die Vorspannung auf dem longitudinal translatierenden Arretierelement (806) zu überwinden, wenn er betätigt wird, um die Gelenkverbindung (702) zu entriegeln.

11. Chirurgisches Instrument (200) nach Anspruch 1, **dadurch gekennzeichnet, dass** der längliche Schaft (204) eine Rahmenanordnung (210) umfasst, umschlossen durch eine longitudinal gleitbar aufgenommene Verschlussmanschettenanordnung (212);
der Endeffektor (12) eine Klammeranbringanordnung (12) umfasst, welche einen länglichen Kanal (18) umfasst, eine Klammerkassette (20), in Eingriff genommen im länglichen Kanal (18), und einen Amboss (22), schwenkbar befestigt am länglichen Kanal (18) und der Klammerkassette (20) eine Klammerausformungsfläche bietend;
wobei der elektroaktive Polymerbetätiger (400) die Gelenkverbindung der Klammeranbringanordnung (12) herstellt;
und weiterhin einen Griffabschnitt (14) umfassend, der an einem proximalen Ende des länglichen Schafts (16) befestigt ist und wirksam gestaltet ist, um selektiv ein elektrisches Signal an den länglichen Schaft (16) zu übermitteln.

## Revendications

1. Instrument chirurgical (200), comprenant :
■ un effecteur terminal (12) ;
■ une tige allongée (204) ; et
■ un raccord d'articulation pivotant (202) comprenant un premier élément cadre (250) fixé à un élément choisi entre l'effecteur terminal (12) et une extrémité distale de la tige allongée (204), un deuxième élément cadre (236) fixé à l'autre élément parmi l'effecteur terminal (12) et l'extrémité distale de la tige allongée (204), et un raccordement pivotant entre les premier et deuxième éléments (250, 236) ; et un activateur polymère électroactif (400) relié entre les premier et deuxième éléments cadres (250, 236) ;
**caractérisé en ce que** le premier élément cadre (250) comprend une cavité s'ouvrant vers le raccordement pivotant et le deuxième élément cadre (236), le deuxième élément cadre (236) comprenant un premier bras de levier (406, 416) s'étendant dans la cavité, l'actionneur (400) étant fixé entre le bras de levier (406, 416) et le premier élément cadre (250) à travers la cavité.

2. Instrument chirurgical (200) selon la revendication 1, dans lequel l'activateur polymère électroactif (400) comprend un activateur de fibres polymères électroactif (402, 404, 412, 414) configuré fonctionnellement pour se contracter.

3. Instrument chirurgical (200) selon la revendication 1, dans lequel le premier élément cadre (250) comprend un premier tenon supérieur (246) et un premier tenon inférieur (248) fixés de façon à pivoter respectivement par rapport à un deuxième tenon supérieur (238) et un deuxième tenon inférieur (240) du deuxième élément cadre (236), les premier et deuxième tenons supérieurs (246, 238) étant espacés latéralement des premier et deuxième tenons inférieurs (248, 240).

4. Instrument chirurgical (200) selon la revendication 3, dans lequel le premier élément cadre (250) comprend un tube définissant une cavité qui reçoit le deuxième élément cadre (236), au moins l'un des deuxièmes tenons supérieurs et inférieurs (238, 240) comprenant un bras de levier (406, 416) s'étendant dans la cavité, l'activateur polymère électroactif (400) comprenant une paire d'activateurs polymères électro-actifs (402, 404, 412, 414) fixée au bras de levier (404, 416) et des surfaces intérieures latérales respectives (406, 408, 418, 420) du premier élément cadre (250).

5. Instrument chirurgical (602) selon la revendication 3, dans lequel au moins un tenon (608) comprend une partie profilée au niveau périphérique (614) transversale par rapport à un axe d'articulation du raccord d'articulation (600), l'activateur polymère électroactif (400) comprenant un activateur de fibres polymères électroactif (616) fixé à la partie profilée au niveau périphérique (614) et placé sur celle-ci à une extrémité et fixé à l'autre élément cadre (606).

6. Instrument chirurgical (602) selon la revendication 5, dans lequel au moins un tenon (608) comprend une contre partie profilée au niveau périphérique, transversale par rapport l'axe d'articulation du raccord d'articulation (600), l'instrument chirurgical (602) comprenant en outre un contre activateur de fibres polymères électroactif fixé à la partie périphérique de compteur et placé sur celle-ci.

7. Instrument chirurgical (602) selon la revendication 6, dans lequel l'un des tenons supérieurs (246, 238) choisi et l'un des tenons inférieurs (248, 240) choisi comprennent tous deux un contour arrondi comprenant une partie supérieure fixée à la fibre polymère électro-active (616) et comprenant une partie inférieure fixée au contre activateur de fibres électroactif.

8. Instrument chirurgical (200) selon la revendication 3, dans lequel l'effectuer terminal (12) comprend un ensemble d'agrafage et de fixation (12) actionné par une barre de déclenchement (270) et une partie de poignée (14) fixée sur le plan proximal à la barre de déclenchement (270) et configurée de façon fonctionnelle pour assurer un mouvement de déclenchement longitudinal de la barre de déclenchement (270), la tige allongée (204) comprenant en outre un guide de barre de déclenchement (272) soutenant la barre de déclenchement (270) au travers d'un raccord d'articulation articulé (202).

9. Instrument chirurgical (200) selon la revendication 8, dans lequel l'effecteur terminal (12) comprend un canal inférieur (18) configuré fonctionnellement de façon à recevoir une cartouche d'agrafes (20) et à comprendre une enclume supérieure (22) fixée de façon pivotante et une partie de poignée (14) configurée fonctionnellement pour produire un mouvement de fermeture longitudinal, la tige allongée (204) comprenant en outre un ensemble de manchon de fermeture (228) couplé sur le plan proximal à la partie de poignée (14) pour transférer le mouvement de fermeture à un raccordement distal avec l'enclume (22), l'ensemble de manchon de fermeture (228) étant configuré fonctionnellement de façon à pivoter autour d'un axe d'articulation du raccord d'articulation (202) dans une position rétractée et dans une position étendue sur le plan distal.

10. Instrument chirurgical (704) selon la revendication 1, comprenant en outre un mécanisme de verrouillage d'articulation (800) comprenant :
■ un élément de verrouillage se déplaçant longitudinalement (806) fixé à l'un des premier et deuxième éléments cadres (718, 708) et incliné de façon à s'étendre vers l'autre des premier et deuxième éléments cadres (718, 708) et étant en prise avec celui-ci ; et
■ un activateur de polymère électroactif (820, 822) configuré fonctionnellement et placé pour pallier l'inclinaison sur l'élément de verrouillage (806) se déplaçant longitudinalement lorsqu'il est activé pour déverrouiller le raccord d'articulation (702).

11. Instrument chirurgical (200) selon la revendication 1, dans lequel
■ la tige allongée (204) comprend un ensemble cadre (210) intégré dans un ensemble de manchon de fermeture (212) reçu longitudinalement de façon coulissante ;
■ l'effecteur terminal (12) comprenant un ensemble d'application d'agrafe (12) comprenant un canal allongé (18), une cartouche d'agrafes (20) en prise dans le canal allongé (18) et une enclume (22) fixée de façon à pivoter au canal allongé (18) présentant une surface de formation d'une agrafe à la cartouche d'agrafes (20) ;
■ l'activateur de polymère électroactif (400) réalisant une articulation de l'ensemble appliquant une agrafe (12) ;
■ et comprenant en outre une partie de poignée (14) fixée à une extrémité proximale de la tige allongée (16) et configuré fonctionnellement pour communiquer sélectivement un signal électrique à la tige allongée (16).
